# EUROPÄISCHE PATENTANMELDUNG

(11) **EP 0 780 364 A1**
(43) Veröffentlichungstag der Anmeldung: **25.06.1997**
(21) Anmeldenummer: 96119695.3
(22) Anmeldetag: 09.12.1996
(51) Int. Cl.: C07C 231/02, C07C 233/58

(54) **Verfahren zur Herstellung von Cyclopropancarbonsäureamiden**

(30) Priorität: 20.12.1995 DE 19547635
(71) Anmelder: BAYER AG, 51368 Leverkusen (DE)
(72) Erfinder: Sattler, Andreas, Dr., 40229 Düsseldorf (DE)

(57) **Zusammenfassung**

Cyclopropancarbonsäureamide der allgemeinen Formel 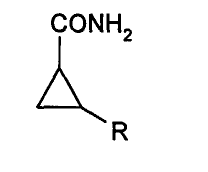 worin
- R: für Wasserstoff, geradkettiges oder verzweigtes C₁-C₄-Alkyl, gegebenenfalls substituiertes Aryl oder gegebenenfalls substituiertes Aralkyl steht,
können direkt aus der entsprechenden Cyclopropancarbonsäure durch Umsetzung mit Ammoniak unter Druck in einem geeigneten organischen Lösungsmittel hergestellt werden.

## Beschreibung

Cyclopropancarbonsäureamide sind wichtige organische Intermediate, so z.B. Cyclopropancarbonsäureamid selbst, das als Vorstufe, zum Beispiel nach Umwandlung in das Cyclopropylamin, einen wichtigen Baustein für die Synthese bedeutender Wirkstoffe im Pharma- und Pflanzenschutzbereich darstellt (z.B.: DE-A 31 42 854). Eine derartige Verwendung erfordert Herstellungsverfahren der Intermediate, die das Produkt nicht nur in relativ hoher Reinheit und hoher Ausbeute liefern, sondern die darüberhinaus durch den Einsatz möglichst weniger Reagenzien und die möglichst direkte Synthese ohne Zwischenstufen zur Vermeidung von Verunreinigungen zu hoher ökonomischer Effizienz beitragen.

Es sind bereits einige Herstellungsverfahren für Cyclopropancarbonsäureamide bekannt; alle gehen jedoch von Derivaten der zugrundeliegenden Cyclopropancarbonsäure aus und benötigen zusätzlich Katalysatoren. So werden in EP-A 0 365 970 C₄-C₈-Alkylester der Cyclopropancarbonsäure, wie z.B. der Isobutylester, mit 35 %iger Natriumisobutanolat/ Isobutanolat-Schmelze als Katalysator und Ammoniak -bei etwa 6 bar und 100°C zum Amid umgesetzt. Man erhält das Cyclopropancarbonsäureamid in einer Ausbeute von ca. 88 %. Durch das bei der Reaktion freiwerdende Isobutanol ist das nach der Umsetzung erhaltene Reaktionsgemisch rührbar und gut filtrierbar. Das Verfahren ist nur für Ester der Cyclopropancarbonsäure verwendbar, darüberhinaus nur für höhere Alkylester. Ferner wird immer Butylat als Katalysator in Mengen um 10 Mol-% bis 20 Mol-% benötigt, uni die Ester erfolgreich zu amidieren. In EP 0 662 470 wird ein sehr ähnliches Verfahren für C₁-C₃-Alkylester der Cyclopropancarbonsäure beschrieben, bei dem beispielsweise der Methylester mit 30 %iger Natriummethylat/Methanol-Lösung als Katalysator und Ammoniak bei etwa 1 bis 3 bar und 60 bis 100°C zum Amid umgesetzt wird. Nach Abdestillieren des Methanols erhält man das Cyclopropancarbonsäureamid in einer Ausbeute von 85 bis 90 %. Durch rechtzeitigen Abbruch der Reaktion bei einem Umsatz von etwa 80 % wird hier das nach der Umsetzung erhaltene Reaktionsgemisch rührbar und gut filtrierbar gehalten. Auch dieses Verfahren erfordert als Reaktanden Ester, auch hier wird Katalysator (Methylat) in nicht unerheblicher Menge von 10 Mol-% bis 30 Mol-% benötigt, uni die Ester erfolgreich zu amidieren. Beide Verfahren liefern also Cyclopropancarbonsäureamid zwar in hohen Ausbeuten und hoher Reinheit, sind jedoch auf Ester als reaktive Derivate der Cyclopropancarbonsäure beschränkt, erfordern darüberhinaus nicht unerhebliche Katalysatormengen und benötigen z.T. Aufarbeitungsprozeduren, die Destillationen und Filtrationen erfordern.

Seit langem literaturbekannt (Dalle, *Centralblatt* **1902** *I*, 913/Kishner, *Centralblatt* **1905** *I*, 1704/Mazur et al., *J Am. Chem. Soc* **1959**, *81*, 4390) sind direkte Umsetzungen der Cyclopropancarbonsäurechloride mit Ammoniak unter Abspaltung von Salzsäure in basischen Lösungsmitteln. Die Carbonsäurechloride werden jedoch in aller Regel zunächst aus den Carbonsäuren durch Umsetzung mit Thionylchlorid oder Phosgen gewonnen. Die Umsetzung der Säurechloride erfordert Hilfsbasen oder mindestens doppelt molare Mengen an Ammoniak. Von Nachteil ist bei diesen Verfahren häufig auch die Entstehung großer Salzrückstände durch die Neutralisationsreaktion.

Auch für einige wenige substituierte Cyclopropancarbonsäureamide sind Herstellungsverfahren wie die oben für Cyclopropancarbonsäureamid dargestellten beschrieben.

Es ist bislang kein Verfahren zur Direktamidierung von Cyclopropancarbonsäure oder substituierten Cyclopropancarbonsäuren mit Ammoniak bekannt, weder mit noch ohne Verwendung von Katalysatoren.

Aufgabe der Erfindung war es also, Cyclopropancarbonsäureamid nicht über eine reaktivere Zwischenstufe wie einen Ester oder ein Säurechlorid, sondern direkt aus der Cyclopropancarbonsäure durch Amidierung mit Ammoniak in hohen Ausbeuten zu erhalten, um optimale Raum/Zeit-Ausbeuten zu erreichen. Dabei sollten keine Katalysatoren Anwendung finden, eine hohe Reinheit des Produkts erreicht werden und die Amidierung unter Druck und Temperaturbedingungen geführt werden, die einer technischen Realisierbarkeit nicht im Wege stehen. Ein derartiges Verfahren wäre hoch effizient und insbesondere ökonomisch den bisherigen Verfahren weit überlegen.

Es wurde nun überraschenderweise ein Verfahren zur Herstellung von Cyclopropancarbonsäureamiden gefunden, welches direkt von den entsprechenden Cyclopropancarbonsäuren ausgehend die gewünschten Verbindungen in hoher Reinheit und mit sehr guten Raum/Zeit-Ausbeuten liefert. Eine Aktivierung der Cyclopropancarbonsäuren durch Derivatisierung zum Ester oder Säurechlorid ist nicht erforderlich.

Die vorliegende Erfindung betrifft ein Verfahren zur Herstellung von Cyclopropancarbonsäureamiden der allgemeinen Formel 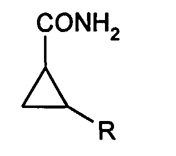 worin
- R: für Wasserstoff, geradkettiges oder verzweigtes C₁-C₄-Alkyl, gegebenenfalls substituiertes Aryl oder gegebenenfalls substituiertes Aralkyl steht,
durch Umsetzung der entsprechenden Cyclopropancarbonsäure mit Ammoniak bei einem Druck von 1 bis 300 bar in Gegenwart eines organischen Lösungsmittels.

Als Substituenten für die gegebenenfalls substituierten Aryl- und Aralkylreste in der Definition von R kommen insbesondere Halogen wie Fluor, Chlor, Brom sowie Nitro, Cyano, COOH und COOC₁-C₄-Alkyl in Frage.

Unter Arylresten sind insbesondere Phenyl und Naphthyl zu verstehen, Arakyl steht vorzugsweise für Phenyl-C₁-C₄-Alkyl oder Napthyl-C₁-C₄-Alkyl, insbesondere Benzyl.

Das nach der Reaktion ausgefallene Cyclopropancarbonsäureamid kann sehr einfach durch Filtration in hohen Ausbeuten isoliert werden. Das nach der Umsetzung erhaltene Reaktionsgemisch ist rührbar, eine einfache Filtration stellt die gesamte Aufarbeitung dar. Ausbeuteverluste wie bei der Esteramidierung nach EP 0 662 470 oder die zwangsläufige Anwesenheit bestimmter bei der Esteramidierung entstehender Alkohole als Additive wie bei der EP-A 0 365 970 treten bei einer Arbeitsweise gemäß dem erfindungsgemäßen Verfahren nicht auf. Die Raum/Zeit-Ausbeute ist deutlich höher als bei den erwähnten Alternativverfahren, da nur Wasser und keine höhermolekularen Alkohole oder HCl bei der Umsetzung des Substrats zum Cyclopropancarbonsäureamid abgespalten wird.

Das erfindungsgemäße Verfahren wird bevorzugt in Abwesenheit von Katalysatoren durchgeführt. In bestimmten Fällen kann jedoch die Verwendung eines Katalysators von Vorteil sein. Als Katalysatoren der Dehydratisierung kommen dabei insbesondere in Frage: Salze von Alkali- und Erdalkalimetallen mit anorganischen Säuren wie z.B. Magnesiumsulfat, Natriumsulfat, Magnesiumchlorid, Calciumchlorid, Calciumcarbonat oder aber Alkali- und Erdalkaliborate, ferner Oxide von Haupt- und Nebengruppenmetallen wie z.B. Aluminiumoxid, Titandioxid oder Zirconoxid, sowie organische Phosphorigsäurederivate wie z.B. Triethylphosphit.

Wenn dem Reaktionsgemisch ein Katalysator zugesetzt wird, so geschieht dies bevorzugt in Mengen von 5 Mol-% bis 100 Mol-% bezogen auf die gegebenenfalls substituierte Cyclopropancarbonsäure.

Zur Durchführung des erfindungsgemäßen Verfahrens können nahezu alle gängigen, inerten organischen Lösungsmittel oder deren Gemische verwendet werden, wie etwa aromatische oder aliphatische Kohlenwasserstoffe, die auch halogeniert sein können, so z.B. Xylole, Toluol, Benzol, Alkylaromaten, Tetralin, Ligroin, Petrolether, Chlorbenzol, Methylenchlorid, Chloroform, Ether wie z.B. Diethylether, Dibutylether oder THF sowie polar-aprotische Lösungsmittel, wie z.B. DMF, DMSO, Sulfolan oder N-Methylpyrrolidon, bevorzugt werden eingesetzt Xylole, Toluol, THF, DMF oder DMSO, besonders bevorzugt verwendet werden Xylole oder Toluol.

Im allgemeinen werden Lösungen mit einem Gehalt von 5-80 Gew.-% an gegebenenfalls substituierter Cyclopropancarbonsäure amidiert, vorzugsweise Lösungen mit einem Gehalt von 30-60 Gew.-%, ganz besonders bevorzugt mit einem Gehalt von 40-50 Gew.-%.

Das erfindungsgemäße Verfahren kann in einem sehr weiten Ammoniakdruckbereich von beispielsweise 1 bis 300 bar durchgeführt werden. Im allgemeinen arbeitet man bei Ammoniakdrücken von 1-50 bar, vorzugsweise von 10-30 bar, ganz besonders bevorzugt von lediglich 15-20 bar.

Die Amidierung wird im allgemeinen in einem Temperaturbereich von 50°C bis 300°C durchgeführt, bevorzugt wird der Bereich zwischen 100°C und 200°C, besonders bevorzugt zwischen 160°C und 190°C.

Ein besonderer Vorteil des erfindungsgemäßen Verfahrens ist darüberhinaus eine unter den als bevorzugt angegebenen milden Druck- und Temperaturbedingungen kurze Reaktionszeit, die beispielweise zwischen 3 und 24 Stunden liegt, vorzugsweise zwischen 6 und 12 Stunden.

Ein weiterer Vorteil des erfindungsgemäßen Verfahrens ist die einfache Isolierbarkeit des Amids durch Filtration aus der nach der Umsetzung gebildeten, rührbaren Suspension, und zwar trotz eines nahezu vollständigen Umsatzes.

Bei der Durchführung des erfindungsgemäßen Verfahrens wird im allgemeinen so vorgegangen, daß die gegebenenfalls substituierte Cyclopropancarbonsäure zusammen mit dem Lösungsmittel oder dem Lösungsmittelgemisch in einem Autoklaven vorgelegt wird, gegebenenfalls unter Zusatz eines Katalysators, und anschließend nach Verschließen des Autoklaven bei Raumtemperatur Ammoniak zudosiert wird. Danach heizt man auf die gewünschte Reaktionstemperatur auf, wobei sich der Arbeitsdruck einstellt, der vorzugsweise innerhalb der ersten Stunden der Reaktionszeit durch Zugabe geringer Mengen Ammoniak gehalten wird. Nach Beendigung der Umsetzung wird der Autoklav abgekühlt und entspannt und das Amid durch Filtration isoliert.

Das erfindungsgemäße Verfahren wird durch die nachfolgenden Beispiele näher erläutert

### Beispiel 1

### Cyclopropancarbonsäureamid

In einem 0.7 l Autoklaven werden 108.6 g 95 %ige Cyclopropancarbonsäure (1.2 mol) in 150 ml Xylol vorgelegt. Es werden 65 ml Ammoniak zugegeben, dann heizt man auf 180°C auf, wobei sich ein Innendruck von 20.5 bar einstellt. Nach einer Stunde bei der obigen Temperatur ist der Druck auf 19 bar abgefallen und es werden 5 ml NH₃ nachdosiert. Nach einer weiteren Stunde werden erneut 7 ml Ammoniak nachgesetzt, anschließend wird noch zehn Stunden bei 180°C und 20 bar gerührt. Man läßt abkühlen und entspannt den Autoklaven. Die gut rührbare Suspension wird ausgenommen, das ausgefallene Cyclopropancarbonsäureamid abgesaugt und mit 20 ml Xylol nachgewaschen. Nach der Trocknung des Produkts im Vakuum bei 70°C erhält man 92.8 g Cyclopropancarbonsäureamid in einer Reinheit von 96.5 %.

### Beispiel 2

### cis/trans-2-Methylcyclopropancarbonsäureamid

In einem 50 ml Autoklaven werden 15 g 98 %ige cis/trans-2-Methylcyclopropancarbonsäure (0.147 mol) in 15 ml Xylol vorgelegt. Es werden 10 ml Ammoniak zugegeben, dann heizt man auf 180°C auf, wobei sich ein Innendruck von 20 bar einstellt. Nach einer Stunde bei der obigen Temperatur ist der Druck auf 19.3 bar abgefallen und es werden 5 ml NH₃ nachdosiert, anschließend wird noch zehn Stunden bei 180°C und 20 bar gerührt. Man läßt abkühlen und entspannt den Autoklaven. Die gut rührbare Suspension wird ausgenommen, das ausgefallene cis/trans-methylcyclopropancarbonsäureamid abgesaugt und mit 10 ml Xylol nachgewaschen. Nach der Trocknung des Produkt im Vakuum bei 70°C erhält man 11.1 g cis/trans-2-Methylcyclopropancarbonsäureamid (0.112 mol).

## Patentansprüche

1. Verfahren zur Herstellung von Cyclopropancarbonsäureamiden der allgemeinen Formel 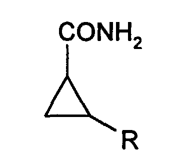 worin
R für Wasserstoff, geradkettiges oder verzweigtes C₁-C₄-Alkyl, gegebenenfalls substituiertes Aryl oder gegebenenfalls substituiertes Aralkyl steht,
dadurch gekennzeichnet, daß die entsprechende Cyclopropancarbonsäure mit Ammoniak bei Ammoniakdrücken von 1 bis 300 bar in Gegenwart eines organischen Lösungsmittels zum Amid umgesetzt wird.

2. Verfahren gemäß Anspruch 1, dadurch gekennzeichnet, daß in Abwesenheit eines Katalysators gearbeitet wird.

3. Verfahren gemäß Ansprüchen 1 und 2, dadurch gekennzeichnet, daß als organisches Lösungsmittel mindestens ein Lösungsmittel aus der Gruppe bestehend aus aromatischen und aliphatischen Kohlenwasserstoffen, die jeweils gegebenenfalls halogeniert sind, Ethern und polar-aprotischen Lösungsmitteln eingesetzt wird.

4. Verfahren gemäß Ansprüchen 1 bis 3, dadurch gekennzeichnet, daß als Lösungsmittel Xylole oder Toluol oder ein beliebiges Gemisch dieser Lösungsmittel eingesetzt wird.

5. Verfahren gemäß Ansprüchen 1 bis 4, dadurch gekennzeichnet, daß Lösungen der entsprechenden Cyclopropancarbonsäure in dem organischen Lösungsmittel mit einem Gehalt von 5-80 Gew.-%, vorzugsweise 30-60 Gew.-% und insbesondere 40-50 Gew.-% mit Ammoniak umgesetzt werden.

6. Verfahren gemäß Ansprüchen 1 bis 5, dadurch gekennzeichnet, daß man bei Ammoniakdrücken von 1-50 bar, vorzugsweise von 10-30 bar, ganz besonders bevorzugt von 15-20 bar arbeitet.

7. Verfahren gemäß Ansprüchen 1 bis 6, dadurch gekennzeichnet, daß die Umsetzung mit Ammoniak in einem Temperaturbereich von 50°C bis 300°C, bevorzugt von 100°C bis 200°C und besonders bevorzugt von 160°C bis 190 °C durchgeführt wird.

8. Verfahren gemäß Ansprüchen 1 bis 7, dadurch gekennzeichnet, daß das Amid durch Filtration isoliert wird.
